# EUROPÄISCHE PATENTANMELDUNG

(11) **EP 2 340 759 A1**
(43) Veröffentlichungstag der Anmeldung: **06.07.2011**
(21) Anmeldenummer: 10015302.2
(22) Anmeldetag: 04.12.2010
(51) Int. Cl.: A61B 1/018, A61B 17/00, A61B 17/29

(54) **Endoskopisches Instrument**

(30) Priorität: 29.12.2009 DE 102009060718
(71) Anmelder: Richard Wolf GmbH, 75438 Knittlingen (DE)
(72) Erfinder: Heimberger, Rudolf, 75038 Oberderdingen (DE); Lambertz, Matthias, Dipl.-Ing., 75015 Bretten (DE)
(74) Vertreter: Hemmer, Arnd

(57) **Zusammenfassung**

Die Erfindung betrifft ein endoskopisches Instrument mit einem länglichen Leitelement (2), welches zumindest eine sich vom proximalen (8) zum distalen Ende (10) erstreckende Leitschiene (6) aufweist, und zumindest einem Arbeitsinstrument (14), welches mit einem zu der Leitschiene passenden Eingriffselement (12) versehen ist, das derart mit der Leitschiene in Eingriff bringbar ist, dass das Arbeitsinstrument entlang der Leitschiene verschiebbar ist.

## Beschreibung

Die Erfindung betrifft ein endoskopisches Instrument.

Grundlage neuartiger endoskopischer Verfahren, wie Ein-Port-Techniken oder NOTES (Natural Orifice Translumenal Endoscopic Surgery) ist, den Zugang zum Operationsfeld möglichst atraumatisch und klein zu halten. Um therapeutisch arbeiten zu können, ist es jedoch erforderlich mehrere Instrumente multifunktional gleichzeitig einzusetzen. Je nach Anzahl und Ausführung dieser zu verwendenden Instrumente (z. B. Greifzangen, Scheren, Dissektoren, HF-Sonden, Ultraschall, Laser, Spülung/Saugung, rotierende Messer, Bildgebung, u. s. w.) ergibt sich jedoch ein mehr oder weniger großer Gesamtdurchmesser des Systems, wodurch dann wiederum eine gewisse Mindestgröße für den Zugang erforderlich ist, was dem Ziel, den Zugang möglichst klein zu halten, zuwiderläuft.

Es ist daher Aufgabe der Erfindung ein endoskopisches Instrument bzw. ein System von endoskopischen Instrumenten dahingehend zu verbessern, dass das Gesamtsystem durch eine relativ kleine Körperöffnung im Operationsfeld platziert werden kann.

Diese Aufgabe wird durch ein endoskopisches Instrument mit den im Anspruch 1 angegebenen Merkmalen gelöst. Bevorzugte Ausführungsformen ergeben sich aus den Unteransprüchen, der nachfolgenden Beschreibung sowie den beigefügten Figuren.

Der Grundgedanke der Erfindung liegt in der Erkenntnis, dass viele in der Endoskopie verwendete Arbeitsinstrumente für die eigentlichen Werkzeuge am distalen Ende eine größere Querschnittsfläche erfordern, als für den sich weiter distalwärts erstreckenden Schaft. D. h. der Schaft kann wesentlich dünner als das distale Ende ausgebildet werden. Ein Gesamtsystem bestehend aus mehreren derartigen Instrumenten kann durch einen vergleichsweise kleinen Zugang geführt werden, wenn die Instrumente nicht alle gleichzeitig, sondern nacheinander durch den Zugang geführt werden, so dass vorzugsweise immer nur ein Instrument mit seinem im Querschnitt größten Bereich gleichzeitig den Zugang passieren muss.

Erfindungsgemäß wird nun ein endoskopisches Instrument bzw. ein System von endoskopischen Instrumenten bereitgestellt, welches es ermöglicht, mehrere entsprechend ausgebildete Arbeitsinstrumente relativ zu einander zu fixieren und definiert nacheinander in das Operationsgebiet vorzuschieben, so dass nicht alle Arbeitsinstrumente gleichzeitig mit ihrem größten Querschnitt den Zugang passieren müssen. Das erfindungsgemäße endoskopische Instrument weißt hierzu ein längliches Leitelement auf. Dieses Leitelement dient zur Befestigung und zum Führen der Arbeitsinstrumente, wobei insbesondere die Arbeitsinstrumente entlang dem Leitelement in das Operationsgebiet vorgeschoben werden können. Das längliche Leitelement stellt somit das Kernelement des erfindungsgemäßen Instrumentes bzw. Instrumentensystems dar. Das längliche Leitelement weist zumindest eine sich vom proximalen bis zum distalen Ende erstreckende Leitschiene auf. Darüber hinaus ist zumindest ein Arbeitsinstrument vorgesehen, welches ein Eingriffselement aufweist, das passend zu der Leitschiene ausgebildet ist, so dass es mit der Leitschiene in Eingriff treten kann. Das Eingriffselement ist dann entlang der Leitschiene verschiebbar, so dass das gesamte Arbeitsinstrument, nachdem es mit dem Eingriffselement in die Leitschiene eingeführt worden ist, entlang der Leitschiene vom proximalen Ende her distalwärts verschoben werden kann, um das Arbeitsinstrument in das Operationsgebiet vorzuschieben.

Bei einer Operation wird erfindungsgemäß somit zunächst das Leitelement durch den Zugang in das Operationsgebiet vorgeschoben und wie gewünscht platziert. Anschließend werden ein oder mehrere Arbeitsinstrumente entlang einer oder mehrere Leitschienen an dem Leitelement in das Operationsgebiet nacheinander vorgeschoben. Dadurch, dass die Arbeitsinstrumente über die Eingriffselemente mit der Leitschiene in Eingriff sind, werden die einzelnen Arbeitsinstrumente definiert an dem Leitelement gehalten und darüber hinaus definiert vorgeschoben. Entsprechend können die Arbeitsinstrumente dann auch nacheinander wieder entlang der Leitschienen proximalwärts zurückgezogen und aus dem Operationsgebiet entnommen werden. Bevorzugt sind mehrere Leitschienen an dem Leitelement vorgesehen, so dass mehrere Arbeitsinstrumente in definierten Positionen zueinander über das Leitelement fixiert werden können und an diesem nacheinander in das Operationsgebiet vorgeschoben werden können.

Zumindest eines der Arbeitsinstrumente weist in Längsrichtung gesehen einen Mittelabschnitt auf, welcher einen kleineren Querschnitt als ein distaler Abschnitt des Instruments aufweist. Wie bereits oben erläutert, ist es beispielsweise bei Zangen und Schneidelementen so, dass diese am distalen Ende, im Querschnitt bezogen auf die Instrumentenlängsachse, mehr Raum beanspruchen, um dort Greif- und/oder Schneidelemente bewegen zu können. Der sich ausgehend von diesem eigentlichen Arbeitsinstrumenten proximalwärts erstreckende Schaft kann wesentlich dünner ausgebildet sein, d. h. einen kleineren Querschnitt aufweisen. Auch andere Arbeitsinstrumente, wie Bildübertragungssysteme, Endoskopoptiken, etc. benötigen häufig am distalen Ende eine gewisse Mindestgröße, um die eigentlichen Arbeitsinstrumente, Objektive, Öffnungen oder Fenster funktional so anordnen zu können, dass optimale Operationsbedingungen geschaffen werden. Die sich proximalwärts erstreckenden Schäfte können häufig jedoch wesentlich dünner ausgestaltet werden, da in diesen nicht so viel Raum benötigt wird, um die im Inneren des Schaftes erforderlichen Systeme, beispielsweise Betätigungsstangen, Lichtleiter, optische Systeme, Spülkanäle, elektrische Leitungen etc. aufnehmen zu können. Daher wird dann im Schaftbereich des Arbeitsinstrumentes dieses möglichst dünn ausgestaltet, so dass auch mehrere Arbeitsinstrumente gemeinsam durch einen möglichst kleinen Zugang hindurchführen können. Um die im Querschnitt erweiterten distalen Enden ebenfalls durch diesen Zugang zuführen zu können, werden die Instrumente, wie oben beschrieben, nacheinander entlang dem Leitelement vorgeschoben, so dass vorzugsweise immer nur eine distale Erweiterung eines Arbeitsinstrumentes gleichzeitig die engste Stelle des Zuganges passieren muss.

Wie oben bereits ausgeführt, weist das Leitelement bevorzugt mehrere Leitschienen auf, wobei diese sich weiter bevorzugt parallel zu einander erstrecken. Besonders bevorzugt sind die mehreren Leitschienen gleichmäßig über den Außenumfang des Leitelementes verteilt angeordnet, so dass mehrere Arbeitsinstrumente um den Außenumfang des Leitelementes verteilt angeordnet werden können. Durch diese Anordnung wird ein möglichst großer Abstand zwischen den einzelnen Arbeitsinstrumenten bereitgestellt, so dass diese ohne miteinander zu kollidieren oder ohne sich zu behindern, entlang der Leitschienen in das Operationsgebiet vorgeschoben werden und dort in der vorgesehenen Weise für eine Operation verwendet werden können. Bevorzugt sind alle Leitschienen gleich ausgebildet. Dies hat den Vorteil, dass die Leitschienen universell verwendet werden können und bevorzugt die Arbeitsinstrumente mittels ihrer Eingriffselemente über beliebige Leitschienen an dem Leitelement vorgeschoben werden können. Dies ermöglicht es dem Operateur, an dem Leitelement die einzelnen Arbeitsinstrumente in der von ihm gewünschten Weise zu platzieren. Die regelmäßige Verteilung der Leitschienen über den Außenumfang des Leitelementes unterstützt diesen Vorteil, da bei dieser Anordnung bevorzugt alle Leitschienen den gleichen Abstand bzw. Winkelabstand zueinander haben und somit beliebig verwendet werden können. Es ist jedoch auch vorstellbar, die Leitschienen unterschiedlich auszubilden oder unterschiedlich weit voneinander beabstandet an dem Leitelement anzuordnen, um eine vorgegebene optimierte Platzierung für bestimmte Arbeitsinstrumente relativ zu einander zu ermöglichen.

Das Leitelement kann starr ausgebildet sein, dabei erstreckt sich das Leitelement mit den Leitschienen vorzugsweise gerade. Alternativ ist es jedoch auch vorstellbar, das Leitelement gekrümmt auszubilden. Auch eine flexible und steuerbare Ausgestaltung des Leitelementes mit den Leitschienen ist denkbar.

Die Leitschienen sind weiter bevorzugt als Nuten in der Umfangsfläche oder Wandung des Leitelementes ausgebildet. Dies hat den Vorteil, dass das Leitelement an seinem Außenumfang keine vorstehenden Elemente bzw. Kannten aufweisen muss, welche beim Einführen des Leitelementes zur Verletzung von Gewebe führen könnten.

Entsprechend ist das Eingriffselement vorzugsweise als Eingriffsvorsprung derart ausgebildet, dass er in zumindest eine der Eingriffsnuten eingreifen kann. Wenn bevorzugt alle Eingriffsnuten gleich ausgebildet sind, sind weiter bevorzugt auch alle Eingriffsvorsprünge gleich ausgebildet, so dass die Arbeitsinstrumente beliebig in die Eingriffsnuten eingesetzt werden können. Alternativ ist es jedoch auch denkbar, bestimmte Eingriffsnuten und Eingriffsvorsprünge so zu dimensionieren bzw. von ihrer Form her zu gestalten, dass ein Eingriffsvorsprung nur in eine vorbestimmte Eingriffsnut einsetzbar ist. So kann sichergestellt werden, dass ein bestimmtes Arbeitsinstrument nur in eine bestimmte Eingriffsnut eingesetzt wird und dass bestimmte Arbeitsinstrumente in vorgegebener Weise zu einander positioniert werden.

Weiter bevorzugt sind die zumindest eine Leitschiene und das passende Eingriffselement derart ausgestaltet, dass das Eingriffselement von der Leitschiene in Richtung quer zur Längsachse der Leitschiene hintergriffen wird oder dass das Eingriffselement die Leitschiene in einer Richtung quer zur Längsachse der Leitschiene umgreift. Auf diese Weise wird eine Sicherung bzw. Befestigung in der Richtung quer zur Längsrichtung der Leitschiene erreicht, so dass das Eingriffselement und das mit ihm verbundene Arbeitsinstrument relativ zu der Leitschiene nur in Richtung deren Längsachse bewegt werden kann, nicht jedoch quer hierzu. Insbesondere wird verhindert, dass das Eingriffselement in einer Richtung quer zur Längsachse der Leitschiene, d. h. insbesondere normal zur Längsachse der Leitschiene von dieser abgezogen werden kann. Auf diese Weise wird verhindert, dass sich Leitschiene und Arbeitsinstrumente während der Operation trennen können. Eine Trennung ist bevorzugt ausschließlich in der Weise möglich, dass das Eingriffselement am proxmalen Ende der Leitschiene von dieser in Längsrichtung abgezogen wird bzw. dass das Eingriffselement in proximaler Richtung aus der Leitschiene herausgezogen wird.

Wenn die Leitschiene als Eingriffsnut ausgebildet ist, weist die Eingriffsnut vorzugsweise an ihrer Außenseite einen offenen Spalt auf, durch welchen das Eingriffselement in die Eingriffsnut eingreift. Im Querschnitt gesehen erweitert sich dieser Spalt im Inneren und korrespondierend auch das Eingriffselement. Beispielsweise können Spalt- und Eingriffselement im Querschnitt im Wesentlichen T-förmig ausgebildet sein. Auf diese Weise wird eine Führung in Längsrichtung der Leitschiene erreicht und verhindert, dass das Eingriffselement aus der Leitschiene quer zur Längsrichtung entnommen werden kann. In besonderen Ausführungsformen ist des denkbar, Leitschiene und Eingriffselement so auszubilden, dass das Eingriffselement in oder um die Leitschiene in einem gewissen Winkelbereich um deren Längsachse schwenk- bzw. drehbar ist. So kann ein Arbeitsinstrument, welches fest mit dem Eingriffselement verbunden oder einstückig mit diesem ausgestaltet ist, in einem gewissen Winkelbereich um die Längsachse der Leitschiene verschwenkt werden.

Das zumindest eine Arbeitsinstrument ist bevorzugt ein Greifinstrument, ein Schneidinstrument, ein HF-Instrument, ein Saug- oder Spülkanal, ein Bildübertragungssystem und/oder eine Beleuchtungseinrichtung. Auch ein Endoskop kann als Arbeitsinstrument angesehen werden. Ein Bildübertragungssystem kann ein Video-System sein oder auch ein optisches System zur direkten Bildübertragung, gegebenenfalls unter Verwendung von Lichtleitern. Ferner ist zu verstehen, dass die vorgenannte Aufzählung von Instrumenten nicht abschließend ist, vielmehr können alle denkbaren Arten von Instrumenten, welche für eine endoskopische Operation benötigt werden, als Arbeitsinstrumente an dem Leitelement befestigt und vorgeschoben werden.

Darüber hinaus kann auch das Leitelement in seinem Inneren zumindest einen sich vom proximalen zum distalen Ende erstreckenden Kanal aufweisen. Ein solcher Kanal kann beispielsweise als Saug- oder Spülkanal eingesetzt werden, darüber hinaus jedoch auch zur Zuführung bzw. Aufnahme weiterer Arbeitsinstrumente Verwendung finden. Weiter bevorzugt kann im Inneren des Leitelementes ein Betrachtungs- und/oder Beleuchtungssystem angeordnet sein. Hierbei kann es sich um eine herkömmliche Endoskopoptik, ein Video-System oder ein System unter Verwendung von Lichtleitern zur Bildübertragung und/oder Beleuchtung handeln. Die Anordnung eines optischen Systems im Inneren des Leitelementes hat den Vorteil, dass bei einem Einführen bzw. Vorschieben des Leitelementes dieses optische System zur Beobachtung Verwendung finden kann, so dass das Leitelement unter visueller Kontrolle sicher im Operationsgebiet platziert werden kann.

Nachfolgend wird die Erfindung beispielhaft anhand der beigefügten Figuren beschrieben. In diesen zeigt:
- Fig. 1: eine perspektivische Ansicht eines Leitelementes eines erfindungsgemäßen endoskopischen Instrumentes,
- Fig. 2: das Leitelement gemäß Fig. 1 mit angesetzten Arbeitsinstrumenten,
- Fig. 3: eine Seitenansicht des Instrumentes gemäß Fig. 2,
- Fig. 4: eine Schnittansicht entlang der Linie IV - IV in Figur 3,
- Fig. 5: eine Schnittansicht entlang der Linie V - V in Figur 3 und
- Fig. 6: eine Schnittansicht entlang der Linie VI - VI in Figur 3.

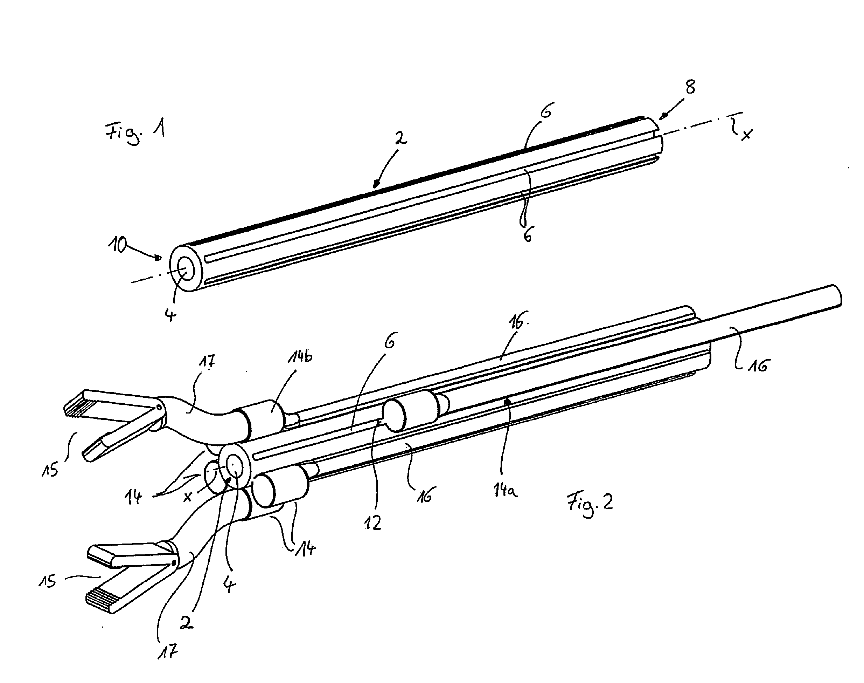

Das Kernelement eines erfindungsgemäßen endoskopischen Instrumentes ist ein Leitelement 2, welches als Einzelteil in Fig. 1 gezeigt ist. Das dort gezeigte Leitelement 2 ist rohrförmig ausgebildet und weist in der Mitte einen zentralen, sich entlang der Längsachse X erstreckenden Kanal 4 auf. Der Kanal 4 kann zur Aufnahme von Arbeitsinstrumenten oder auch eines optischen Systems dienen. Ein solches optisches System in dem Kanal 4 kann beispielsweise dazu dienen, das Leitelement 2 unter visueller Kontrolle in das Operationsgebiet vorzuschieben. Alternativ oder zusätzlich kann der Kanal 4 aber auch als Spül- oder Saugkanal Verwendung finden. Am Außenumfang des Leitelementes 2 sind in dem hier gezeigten Beispiel sechs Leitschienen 6 in Form von Führungsnuten ausgebildet, welche sich parallel zur Längsachse X vom proximalen Ende 8 zum distalen Ende 10 des Leitelementes 2 erstrecken. Während die Leitschienen 6 zum proximalen Ende 8 hin geöffnet sind, erstrecken sie sich nicht ganz bis zum distalen Ende 10 und sind somit zum distalen Ende 10 hin geschlossen ausgebildet.

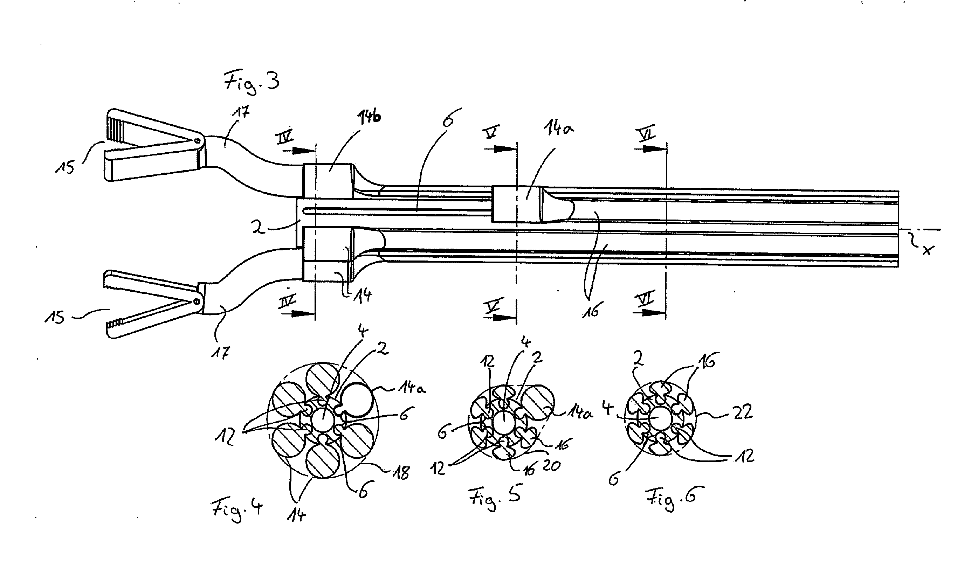

Die Leitschienen 6 haben, wie in den Figuren 4 bis 6 zu erkennen ist, einen kreisförmigen Querschnitt und sind zum Außenumfang des Leitelementes 2 durch einen Spalt geöffnet. In diesen greift ein Eingriffselement 12 in Form eines Eingriffsvorsprunges ein. Dieser weist dabei einen Querschnitt auf, welcher dem Querschnitt der Leitschiene 6 entspricht, so dass ein formschlüssiger Eingriff geschaffen wird und insbesondere das Eingriffselement 12 nur vom proximalen Ende 8 her in die Leitschiene 6 eingeschoben werden kann, aber in radialer Richtung bezogen auf die Längsachse X in dieser gesichert wird, indem die Leitschiene 6 den kreisförmigen Vorsprung des Eingriffselementes 12 umgreift. So wird eine Bewegung quer zur Längsachse X verhindert.

Die Eingriffselemente 12 sind fest bzw. einstückig mit den Arbeitsinstrumenten 14 verbunden bzw. ausgebildet. Auf diese Weise können die Arbeitsinstrumente 14 (14, 14 a, 14 b) jeweils in eine der Leitschienen 6 mit dem Eingriffselement 12 eingeschoben und entlang dieser Leitschiene 6 zum distalen Ende 10 hin entlang dem Leitelement 2 vorgeschoben werden. In Fig. 2 sind fünf Arbeitsinstrumente 14 bereits in der distalen Position gezeigt, während das Arbeitsinstrument 14 a noch in einer Zwischenposition, d. h. während des Vorschubs zum distalen Ende hin dargestellt ist.

Bei den Arbeitsinstrumenten 14 kann es sich um beliebige Instrumente handeln, welche für endoskopische Eingriffe Verwendung finden, beispielsweise Greif-, Schneidinstrumente, Saug- und Spülkanäle, optische Systeme, Endoskope, HF-Instrumente, etc. Beispielhaft sind in den Figuren zwei Arbeitsinstrumente als Zange 15 dargestellt.

Wie in Figuren 2 und 3 zu erkennen ist, weisen die Arbeitsinstrumente 14 an ihrem distalen Ende einen größeren Querschnitt bezogen auf die Instrumentenlängsachse X auf als im Bereich ihres übrigen Schaftes. Wie beispielsweise anhand des Instrumentes 14 b zu sehen ist, ist am distalen Ende ein größerer Querschnitt erforderlich, um hier die gewünschte Funktionalität, in dem Beispiel die Beweglichkeit eines Zangenmauls 15, gewährleisten zu können. Die Schäfte 16, welche sich proximalwärts zum proximalen Ende 8 hin erstrecken, weisen im Querschnitt einen geringeren Querschnitt auf. In diesen Schäften sind lediglich die erforderlichen Versorgungskanäle, Betätigungsstangen und ähnliches angeordnet. Im hier gezeigten Beispiel weisen die Arbeitsinstrumente 14 alle ein äußeres Schaftrohr auf, welches an seinem distalen Ende z. B. eine Zange 15, Schere oder dergleichen aufweisen kann. Im Inneren dieses Schaftrohres kann das eigentliche Arbeitsinstrument wiederum beweglich angeordnet sein, so dass das Arbeitsinstrument proximalwärts und distalwärts bewegt werden kann. Auf diese Weise wird die Beweglichkeit jedes einzelnen Instrumentes während der Operation unabhängig von der Leitschiene 6 sichergestellt. Gleichzeitig ist es auch möglich, die Instrumente innerhalb der äußeren Schaftrohre drehbar auszubilden, so dass auch eine Veränderung der Winkellage jedes einzelnen Arbeitsinstrumentes 14 bzw. des in dem Schaftrohr des Arbeitsinstrumentes 14 angeordneten eigentlichen Instrumentes möglich ist.

Bei denen als Beispiel gezeigten Zangen 15 ist zwischen dem distalen Ende der Schäfte 16 und dem eigentlichen Zangenmaul noch ein Schaftabschnitt 17 angeordnet, welcher im hier gezeigten Beispiel derart geknickt verläuft, dass das Zangenmaul radial nach außen versetzt ist. Dieser Schaftabschnitt 17 kann starr ausgebildet sein, es ist jedoch auch denkbar, dass dieser Schaftabschnitt 17 flexibel bzw. auslenkbar ausgestaltet ist, um das Zangenmaul der Zange 15 im Operationsgebiet zu bewegen bzw. zu positionieren, insbesondere auch in radialer Richtung bezogen auf die Längsachse X positionieren zu können.

Wie in Fig. 4 zu sehen ist, ist der Querschnitt 18 im Bereich des distalen Endes der Gesamtanordnung ziemlich groß. Dort befinden sich die im Querschnitt erweiterten Bereiche der einzelnen Arbeitsinstrumente 14, so hier dass sich ein maximaler Gesamtquerschnitt 18 ergibt. Wie in Figur 6 gezeigt, ergibt sich im Bereich der Schäfte 16 ein deutlich kleinerer Gesamtquerschnitt 22, welcher der minimale Querschnitt des Gesamtinstrumentes bzw. Instrumenten-Systems ist. Erfindungsgemäß ist es nun so, dass die einzelnen Arbeitsinstrumente 14 nicht gleichzeitig durch den Zugang vorgeschoben werden, sondern nacheinander an dem Leitelement 2 in ihrer jeweiligen Leitschiene 6 zum distalen Ende 10 hin und damit ins Operationsgebiet vorgeschoben werden. So ergibt sich, wie in Fig. 5 gezeigt, im Bereich des Zuganges, durch welchen das Instrument in den Körper geführt wird, stets nur ein gegenüber dem minimalen Querschnitt 22 geringfügig vergrößerter Querschnitt 20. Dieser Querschnitt vergrößert sich jeweils nur um das Maß eines einzelnen Arbeitsinstrumentes 14, wie anhand des noch nicht ganz vorgeschobenen Arbeitsinstrumentes 14 a in Figuren 3 und 5 gezeigt ist. Auf diese Weise wird es möglich, eine Anzahl von Arbeitsinstrumenten 14, welche am distalen Ende gemeinsam ein großen Querschnitt 18 definieren, durch eine kleinere Querschnittsfläche 20 nacheinander in das Operationsgebiet vorzuschieben. So kann bei einem sehr kleinen Zugang dennoch das gesamte Instrumenten-System bestehend aus zahlreichen Arbeitsinstrumenten durch einen einzigen Zugang in das Operationsgebiet gebracht werden.

Die Arbeitsinstrumente 14 können in den Leitschienen 6 abgedichtet angeordnet sein. Darüber hinaus kann für den Fall, dass nicht alle Leitschienen 6 mit Arbeitsinstrumenten 14 belegt werden, eine Abdichtung der unbenutzten Leitschienen 6 durch Opturatore möglich sein. Durch solche Abdichtungen wird ein Austreten von die Körperhöhle aufweitenden Körperhöhlengas verhindert.

### Bezugszeichenliste

- 2: - Leitelement
- 4: - Kanal
- 6: - Leitschienen
- 8: - proximales Ende
- 10: - distales Ende
- 12: - Eingriffselement
- 14: - Arbeitsinstrument
- 16: - Schaft
- 17: - Schaftabschnitt
- 18, 20, 22: - Querschnitt
- X: - Längsachse

## Patentansprüche

1. Endoskopisches Instrument mit einem länglichen Leitelement (2), welches zumindest eine sich vom proximalen 8 zum distalen Ende (10) erstreckende Leitschiene (6), und zumindest ein Arbeitsinstrument (14) aufweist, welches mit einem zu der Leitschiene (6) passenden Eingriffselement (12) versehen ist, das derart mit der Leitschiene (6) in Eingriff bringbar ist, dass das Arbeitsinstrument (14) entlang der Leitschiene (6) verschiebbar ist.

2. Endoskopisches Instrument nach Anspruch 1, bei welchem das zumindest eine Arbeitsinstrument (14) in Längsrichtung (X) gesehen einen Mittelabschnitt (16) aufweist, welcher einen kleineren Querschnitt als ein distaler Abschnitt aufweist.

3. Endoskopisches Instrument nach Anspruch 1 oder 2, welches mehrere, sich vorzugsweise parallel zueinander erstreckende Leitschienen (6) aufweist.

4. Endoskopisches Instrument nach einem der vorangehenden Ansprüche, bei welchem die eine oder mehrere Leitschienen (6) als Nuten in der Umfangsfläche des Leitelementes (2) ausgebildet sind.

5. Endoskopisches Instrument nach Anspruch 4 bei welchem das Eingriffselement (12) als Eingriffsvorsprung derart ausgebildet ist, dass er in zumindest eine der Eingriffsnuten (6) eingreifen kann.

6. Endoskopisches Instrument nach einem der vorangehenden Ansprüche, bei welchem die zumindest eine Leitschiene (6) und das passende Eingriffselement (12) derart ausgestaltet sind, dass das Eingriffselement (12) von der Leitschiene (6) in Richtung quer zur Längsachse (X) der Leitschiene (6) hintergriffen wird.

7. Endoskopisches Instrument nach einem der vorangehenden Ansprüche, bei welchem das zumindest eine Arbeitsinstrument (14) ein Greifinstrument, ein Schneidinstrument ein HF-Instrument, ein Saug- oder Spülkanal, ein Bildübertragungssystem und/oder eine Beleuchtungseinrichtung ist.

8. Endoskopisches Instrument nach einem der vorangehenden Ansprüche, bei welchem das Leitelement (2) in seinem Inneren zumindest einen sich vom proximalen zum distalen Ende erstreckenden Kanal (4) aufweist.

9. Endoskopisches Instrument nach einem der vorangehenden Ansprüche, bei welchem im Inneren des Leitelementes (2) ein Betrachtungs- und/oder Beleuchtungssystem angeordnet ist.
